# EUROPEAN PATENT SPECIFICATION

(11) **EP 3 549 430 B1**
(45) Date of publication and mention of the grant of the patent: **23.06.2021**
(21) Application number: 17889134.7
(22) Date of filing: 13.12.2017
(51) Int. Cl.: A01D 34/64, A61B 5/18, A61M 21/02, G05D 1/02, A61B 5/16

(54) **EMOTION IMPROVEMENT DEVICE AND EMOTION IMPROVEMENT METHOD**
GEFÜHLSVERBESSERUNGSVORRICHTUNG UND GEFÜHLSVERBESSERUNGSVERFAHREN
DISPOSITIF D'AMÉLIORATION D'ÉMOTION ET PROCÉDÉ D'AMÉLIORATION D'ÉMOTION

(30) Priority: 27.12.2016 JP 2016253719
(43) Date of publication of application: 09.10.2019
(73) Proprietor: Honda Motor Co., Ltd., Tokyo 107-8556 (JP)
(72) Inventor: HANEDA Satoshi, Wako-shi Saitama 351-0193 (JP); YAMAMURA Makoto, Wako-shi Saitama 351-0193 (JP); UDAGAWA Takamasa, Wako-shi Saitama 351-0193 (JP); FUJIWARA Masato, Wako-shi Saitama 351-0193 (JP); ONODERA Satoshi, Tokyo 107-8556 (JP); KIMATA Ryuichi, Tokyo 107-8556 (JP)
(74) Representative: Beder, Jens
(86) International application number: PCT/JP2017/044774
(87) International publication number: WO 2018/123598

(56) References cited:
- JP-A- 2006 282 115
- JP-A- 2008 054 915
- JP-A- 2008 070 966
- JP-A- 2016 137 203
- JP-B2- 5 050 449
- JP-B2- 5 775 440
- JP-B2- 5 917 841
- US-A1- 2010 134 302
- US-A1- 2014 088 840
- US-A1- 2014 088 840

## Description

### TECHNICAL FIELD

This invention relates to an emotion improving apparatus and an emotion improving method for improving an emotion of users of a working machine.

### BACKGROUND ART

Apparatuses have been known that estimate the emotions of multiple occupants of a vehicle and control the vehicle so that the vehicle improves the emotions (for example, see JP 2016 - 137 200 A). In the case of the apparatus described in JP 2016 - 137 200 A, when occupants start to quarrel with each other during a travel of a vehicle, it blows the maximum amount of cold blast on the occupants through the air outlet of an air-conditioner to give the occupants an opportunity to restore calm.

US 2014 / 0 088 840 A1 discloses a method for operating an agricultural working machine, wherein an emotional state of an operator during an interaction with the working machine via at least one interface is recognized and an operating mode of the working machine is adapted to the emotional state of the operator. The emotional state can be detected by measuring physical data of the operator, such as pulse rate, blood pressure, eye movement or hand temperature.

### DISCLOSURE OF INVENTION

### PROBLEMS TO BE SOLVED BY THE INVENTION

It is desirable to configure a working machine such that if the emotions of users thereof deteriorate, the working machine can improve the emotions. Unfortunately, the apparatus of JP 2016 - 137 200 A is configured to improve the emotions of occupants of a vehicle, and it is difficult to apply the technology of JP 2016 - 137 200 A to a working machine, which is not premised on a ride in a vehicle.

### MEANS FOR SOLVING PROBLEM

An aspect of the present invention is an emotion improving apparatus for improving an emotion improving apparatus for improving an uncomfortable emotion of a user of a working machine, comprising: the working machine comprising at least one of a lawn mower configured to perform a lawn-mowing work while autonomously traveling in a working area and a sprinkler; a biological information acquiring unit configured to acquire a biological information of the user; an uncomfortable emotion determining unit configured to determine whether a predetermined uncomfortable emotion occurs to the user based on the biological information acquired by the biological information acquiring unit; and a working machine controlling unit configured to control the working machine to perform a predetermined operation for improving the uncomfortable emotion to the user when it is determined by the uncomfortable emotion determining unit that the predetermined uncomfortable emotion occurs.

Another aspect of the present invention is an emotion improving method for improving an uncomfortable emotion of a user of a working machine, wherein the working machine comprises at least one of a lawn mower configured to perform a lawn-mowing work while autonomously traveling in a working area and a sprinkler, comprising: acquiring, by means of an biological information acquiring unit, a biological information of the user; determining, by means of an uncomfortable emotion determining unit, whether a predetermined uncomfortable emotion occurs to the user based on the biological information acquired; and controlling, by means of a working machine controlling unit, the working machine to perform a predetermined operation for improving the uncomfortable emotion to the user when it is determined that the predetermined uncomfortable emotion occurs.

### EFFECT OF THE INVENTION

According to the present invention, uncomfortable emotions of users of a working machine can be favorably improved on the basis of the operation of the working machine.

### BRIEF DESCRIPTION OF DRAWINGS

FIG. 1 is a drawing showing a schematic configuration of a working machine including an emotion improving apparatus according to an embodiment of the present invention;
FIG. 2A is a block diagram showing a schematic configuration of the emotion improving apparatus of FIG. 1;
FIG. 2B is a block diagram showing a functional configuration of an ECU of FIG. 2A;
FIG. 3 is a plan view showing an example of a working area for a work by the working machine of FIG. 1;
FIG. 4 is a flowchart showing an example of a process performed by a CPU of FIG. 2A;
FIG. 5 is a diagram showing an example of operation of the emotion improving apparatus according to the embodiment of the present invention;
FIG. 6 is a drawing showing a modification of FIG. 1; and
FIG. 7 is a drawing showing an example for configuring an emotion improving apparatus with multiple working machines.

### DESCRIPTION OF EMBODIMENT

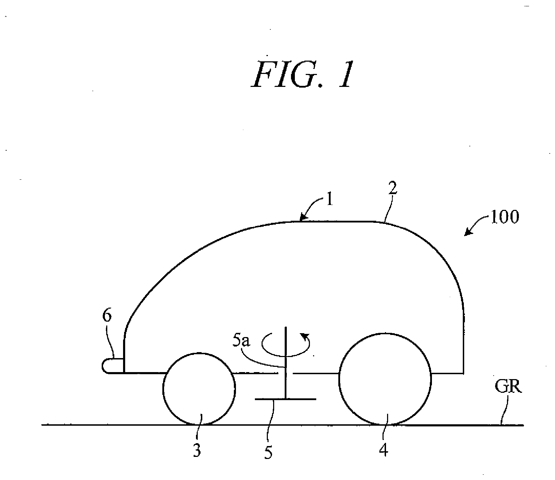

Now, an embodiment of the present invention will be described with reference to FIGs. 1 to 7. An emotion improving apparatus of the present invention can be applied to various types of working machines. In the following embodiment, there will be described an example of application of the emotion improving apparatus of the present invention to a lawn mower that is able to mow the lawn while traveling autonomously.

FIG. 1 is a side view showing a schematic configuration of a working machine (lawn mower) 1 including an emotion improving apparatus 100 according to the embodiment of the present invention. As shown in FIG. 1, the working machine 1 includes a body 2, a pair of left and right front wheels 3, and a pair of left and right rear wheels 4. The working machine 1 has a weight and size such that any user can carry it with his or her hands. In an example, the working machine 1 has an overall length of about 500 mm, an overall width of about 300 mm, and a height of about 300 mm.

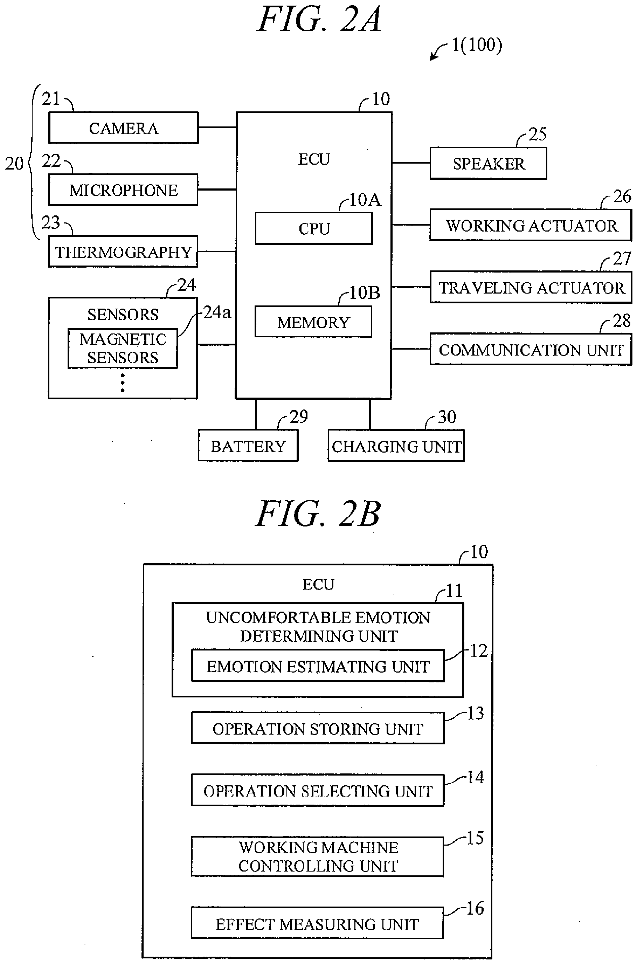

A disc-shaped, lawn mowing blade 5 supported by a rotating shaft 5a protrudes toward the ground (lawn ground) GR from the bottom of the body 2. The working machine 1 is able to mow the lawn by rotating the blade 5 while traveling on the ground GR. The front end of the body 2 is provided with charging terminals 6 for charging a battery (FIG. 2A) mounted on the body 2.

FIG. 2A is a block diagram showing a schematic configuration of the emotion improving apparatus 100 disposed on the working machine 1. As shown in FIG. 2A, the working machine 1 includes an electronic control unit (ECU) 10 and also includes a camera 21, a microphone 22, a thermography 23, sensors 24, a speaker 25, a working actuator 26, a traveling actuator 27, a communication unit 28, a battery 29, and a charging unit 30 that are connected to the ECU 10. All these components are mounted on the body 2. The ECU 10 includes a CPU (processor) 10A, a memory 10B such as ROM or RAM, other peripheral circuits, and the like.

The camera 21, microphone 22, and thermography 23 form a biological information acquiring unit 20 that acquires biological information of users. Specifically, the camera 21 captures images of the upper-bodies of the users, including the faces, and detects the facial expressions or motions of the users; the microphone 22 acquires speeches uttered by the users, and the speeches acquired by the microphone 22 are recognized by a speech recognition unit (not shown) of the ECU 10; and the thermography 23 detects the body temperatures of the users, for example, the surface temperatures of the faces.

The biological information acquiring unit 20 (21 to 23) is used to estimate the emotions of the users. Note that the users include not only a person who is using the working machine 1, but also persons present around the working machine 1. That is, persons recognizable by the working machine 1, in other words, persons around the working machine 1 are referred to as the users herein.

The sensors 24 include a pair of magnetic sensors 24a spaced from each other in the vehicle width direction, and the magnetic sensors 24a detect the magnetic field strength. Although not shown, the sensors 24 also include a Yaw sensor that detects the angular velocity around an axis in the height direction of the working machine 1, a G sensor that detects the acceleration that acts on the working machine 1, an azimuth sensor that detects the azimuth of the working machine 1, a contact sensor that detects whether the working machine 1 has contacted an obstacle, a wheel speed sensor that detects the wheel speed of the left and right rear wheels 4, a GPS sensor that detects the position of the working machine 1, a voltage sensor that detects the remaining voltage of the battery 29, and the like. The speaker 25 outputs a speech to the users. The microphone 22 and speaker 25 allow the working machine 1 to interact with the user.

The working actuator 26 comprises an electric motor coupled to the rotating shaft 5a. The blade 5 is rotationally driven by driving the working actuator 26. The traveling actuator 27 comprises a pair of electric motors that are disposed inside the left and right rear wheels 4 in the left-right direction and independently drive the left and right rear wheels 4. The working machine 1 can be turned in any direction by making a difference in rotation speed between the left and right rear wheels 4.

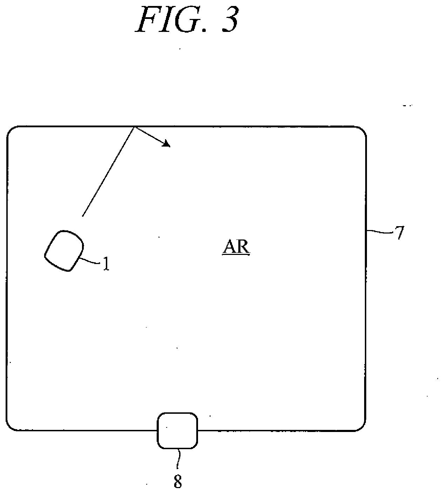

The communication unit 28 includes a transmitting/receiving antenna and a signal processing circuit that processes a signal transmitted or received through the transmitting/receiving antenna. The working machine 1 is able to wirelessly communicate with external devices (e.g., a relay device or server disposed in the same site, mobile terminals carried by the users, etc.) through the communication unit 28. The battery 29 is used as a power supply for supplying power to the electric components of the working machine 1. The charging unit 30 is connected to the charging terminals 6 and battery 29 and stores power supplied from a charging station (FIG. 3) in the battery 29 through the charging terminals 6.

The working machine 1 thus configured works while autonomously traveling within a predetermined working area. FIG. 3 is a plan view showing an example of a working area AR. The working area AR is defined by an area wire 7 previously installed in the garden (e.g., buried at a predetermined depth from the ground), and the travel range of the working machine 1 is defined by the area wire 7. A magnetic field is generated in the working area AR by passing a current through the area wire 7. The magnetic field strength of the working area AR is detected by the magnetic sensors 24a.

The magnetic field strength changes in accordance with the distance from the area wire 7. The ECU 10 (CPU 10A) determines whether the working machine 1 has reached the area wire 7, on the basis of signals from the magnetic sensors 24a. If it determines that the working machine 1 has reached the area wire 7, the ECU 10 outputs control signals to the traveling actuator 27 to turn the working machine 1 toward the inside of the working area AR, as shown by an arrow in FIG. 3. As seen above, the ECU 10 outputs control signals to the traveling actuator 27 in accordance with the signals from the magnetic sensors 24a and thus the working machine 1 autonomously travels within the working area AR. At this time, the ECU 10 outputs control signals also to the working actuator 26 and thus the working machine 1 automatically mows the lawn while traveling within the working area AR.

A charging station 8 for charging the battery 29 is disposed on the area wire 7. If the sensors 24 (e.g., a voltage sensor) detect a voltage shortage of the battery 29 while the working machine 1 works, the ECU 10 outputs control signals to the traveling actuator 27 to return the working machine 1 to the charging station 8, for example, along the area wire 7 and then charges the battery 29. When the charge of the battery 29 is complete, the ECU 10 outputs control signals to the traveling actuator 27 to cause the working machine 1 to leave the charging station 8. The ECU 10 then drives the working actuator 26 to cause the working machine 1 to restart to work.

As described above, the working area AR is set by installing the area wire 7, and the working machine 1 (ECU 10) works while recognizing the working area AR in accordance with the signals from the magnetic sensors 24a. However, the method for setting and recognizing the working area is not limited to that described above. For example, the ECU 10 may receive a beacon signal through the communication unit 28, and the working machine 1 may work in the working area while recognizing the working area using the received signal. Also, a map of the working area may be previously stored in the memory 10B of the ECU 10, and the working machine 1 may work in the working area while detecting its own position using a GPS sensor or the like. That is, the working area may be set using a working area setting unit other than the area wire 7, and the working machine may work while recognizing the working area using a position detector other than the magnetic sensors.

If multiple users (persons around the working machine 1) have uncomfortable emotions in the working area AR due to, for example, a quarrel between them, it is preferred to improve the uncomfortable emotions. In the present embodiment, an attempt is made to improve the uncomfortable emotions using the working machine 1. That is, the working machine 1 automatically works near the users and therefore the users tend to feel familiar with the working machine 1; and for this reason, in the present embodiment, the working machine 1 is configured as follows to provide the users with a predetermined service for improving the uncomfortable emotions if the users have uncomfortable emotions.

FIG. 2B is a block diagram showing the functional configuration of the ECU 10. As shown in FIG. 2B, the ECU 10 includes an uncomfortable emotion determining unit 11, an operation storing unit 13, an operation selecting unit 14, a working machine controlling unit 15, and an effect measuring unit 16. Among these, the uncomfortable emotion determining unit 11, operation selecting unit 14, working machine controlling unit 15, and effect measuring unit 16 are functions performed by the CPU 10A, and the operation storing unit 13 is a function performed by the memory 10B. The uncomfortable emotion determining unit 11 includes an emotion estimating unit 12.

The emotion estimating unit 12 estimates the emotions of the users on the basis of biological information acquired by the biological information acquiring unit 20. For example, using the wheel of emotions of Plutchik, the emotions of the users can be categorized into eight basic emotions (anticipation, joy, acceptance, fear, surprise, sadness, disgust, and anger) and application emotions consisting of adjacent pairs of these emotions. The emotion estimating unit 12 selects one of these categorized emotions, that is, emotion models.

More specifically, the emotion estimating unit 12 first identifies the facial expressions (the mouth angles, etc.) or motions (gestures, etc.) of the users on the basis of image signals from the camera 21, identifies details of speeches uttered by the users or characteristics of the speeches, such as accent or pronunciation, on the basis of speech signals from the microphone 22, and identifies the facial temperatures of the users on the basis of temperature detection signals from the thermography 23. The emotion estimating unit 12 then estimates the emotions of the users by comparing the forms of these identified facial expressions, motions, speech details or characteristics, and facial temperatures with the forms of multiple facial expressions, motions, speech details or characteristics, and facial temperatures previously stored so as to be associated with multiple emotions and determining which of the previously stored forms the identified forms match.

The uncomfortable emotion determining unit 11 converts the emotions of the users estimated by the emotion estimating unit 12 into numerical values. For example, it converts comfortable emotions (positive emotions), such as joy and fun, into positive values and uncomfortable emotions (negative emotions), such as sadness and anger, into negative values. For example, a stronger emotion (a more inner emotion in the wheel of emotions) is given a greater value (absolute value) as a numerical value representing the emotion. Thus, the uncomfortable emotion determining unit 11 obtains the levels of the uncomfortable emotions (e.g., the levels of anger) of the users. If the obtained levels of the uncomfortable emotions reach a predetermined value, the uncomfortable emotion determining unit 11 determines that predetermined uncomfortable emotions to be improved are occurring.

The operation storing unit 13 stores multiple candidate improvement operations for improving uncomfortable emotions. Examples of the improvement operations include an operation in which the working machine 1 traverses between multiple users by driving the traveling actuator 27, an operation in which the working machine 1 travels in a predetermined pattern (e.g., in a zig-zag pattern) around users by driving the traveling actuator 27, an operation in which the working machine 1 mows the lawn in a predetermined pattern (e.g., in a heart pattern) by driving the working actuator 26 and traveling actuator 27, an operation in which the working machine 1 outputs a speech providing a topic or including apology words from the speaker 25, an operation in which the working machine 1 outputs from the speaker 25 an speech inputted through the microphone 22, like a parrot, an operation in which the working machine 1 outputs predetermined music from the speaker 25, an operation in which the working machine 1 performs transmission or placement of a predetermined message email or call to the mobile phones of users through the communication unit 28, and an operation in which the working machine 1 outputs one of past conversations recorded when users had comfortable emotions.

These improvement operations are stored with priorities. An improvement operation expected to improve an uncomfortable emotion to a greater degree is assigned a higher priority.

The operation selecting unit 14 selects an improvement operation to be applied to improve the uncomfortable emotions, from among the improvement operations stored in the operation storing unit 13. For example, it selects an improvement operation having the highest priority. Alternatively, the operation selecting unit 14 may determine an operation to be selected, in accordance with the levels of the uncomfortable emotions obtained by the uncomfortable emotion determining unit 11. For example, if the estimated levels of the uncomfortable emotions are low, the operation selecting unit 14 may select an improvement operation corresponding to the uncomfortable emotions rather than an improvement operation having the highest priority. Or, the operation selecting unit 14 may preferentially select an improvement operation that the working machine 1 can easily perform.

The working machine controlling unit 15 outputs control signals to at least one of the working actuator 26, traveling actuator 27 and speaker 25 so that the working machine 1 performs the improvement operation selected by the operation selecting unit 14, that is, the predetermined operation for improving the uncomfortable emotions.

The effect measuring unit 16 measures the degrees of improvement of the uncomfortable emotions when the working machine 1 performs the improvement operation selected by the operation selecting unit 14 on the basis of the command from the working machine controlling unit 15. Specifically, the emotions of the users on the basis of the biological information acquired by the biological information acquiring unit 20 is estimated, and the effect measuring unit 16 determines whether the levels of the uncomfortable emotions have been improved to a predetermined value or more, for example, whether the levels of the uncomfortable emotions (negative emotions) have become zero, that is, a sufficient uncomfortable emotion improving effect has been obtained.

If the effect measuring unit 16 determines that the degrees of improvement of the uncomfortable emotions made by performing the selected improvement operation are less than the predetermined value, the operation selecting unit 14 lowers the priority of that operation and selects another improvement operation. For example, if an improvement operation having the highest priority is selected and the degrees of improvement of the uncomfortable emotions made by performing this improvement operation are less than the predetermined value, the operation selecting unit 14 lowers the priority of the improvement operation by one level and selects an improvement operation having the second highest priority. Then, the selected improvement operation is performed. Thus, if the effect measuring unit 16 determines that the degrees of improvement of the uncomfortable emotions are the predetermined value or more, the operation selecting unit 14 raises the priority of that operation, that is, updates priorities of the improvement operations stored in the operation storing unit 13.

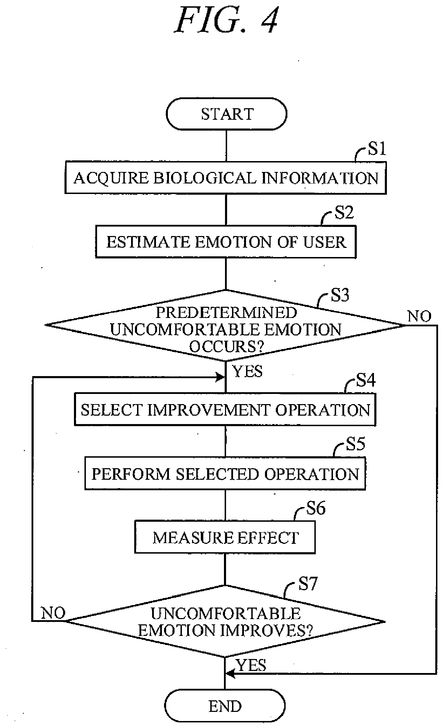

FIG. 4 is a flowchart showing an example of a process performed by the ECU 10 (CPU 10A) in accordance with a program previously stored in the memory 10B. The process shown in this flowchart is started, for example, when the working machine 1 detects a conversation between multiple users using the camera 21 or microphone 22 and repeatedly performed in a predetermined cycle.

First, in step S1, the biological information acquiring unit 20 (camera 21, microphone 22, thermography 23) acquires biological information of the users. Then, in step S2, the emotion estimating unit 12 estimates the emotions of the users on the basis of the acquired biological information. Then, in step S3, the uncomfortable emotion determining unit 11 calculates the levels of the uncomfortable emotions of the users by converting the emotions of the users estimated in step S1 into numerical values. It also determines whether the levels of the uncomfortable emotions reach the predetermined value, that is, predetermined uncomfortable emotions are occurring.

If the determination in step S3 is YES, the process proceeds to step S4; if the determination is NO, the process ends. In step S4, the operation selecting unit 14 selects, for example, an improvement operation having the highest priority from among the improvement operations previously stored in the operation storing unit 13. Then, in step S5, the working machine controlling unit 15 controls the working machine 1 so that the working machine 1 performs the improvement operation selected in step S4. For example, the working machine controlling unit 15 outputs control signals to the traveling actuator 27 so that the working machine 1 performs a predetermined travel operation.

Then, in step S6, the effect measuring unit 16 estimates the emotions of the users on the basis of the biological information acquired by the biological information acquiring unit 20. That is, it measures the uncomfortable emotion improving effect. Then, in step S7, the effect measuring unit 16 determines whether the levels of the uncomfortable emotions have been improved to the predetermined value or more. If the determination in step S7 is NO, the process returns to step S4. In this case, the effect measuring unit 16 lowers the priority of the most recently selected improvement operation (e.g., an improvement operation having the highest priority) by one level and selects another improvement operation (e.g., an improvement operation having the second highest priority) to update priorities of the improvement operations stored in the operation storing unit 13. Hereafter, a similar process is repeated until the determination in step S7 becomes YES. When the determination in step S7 becomes YES, the process ends.

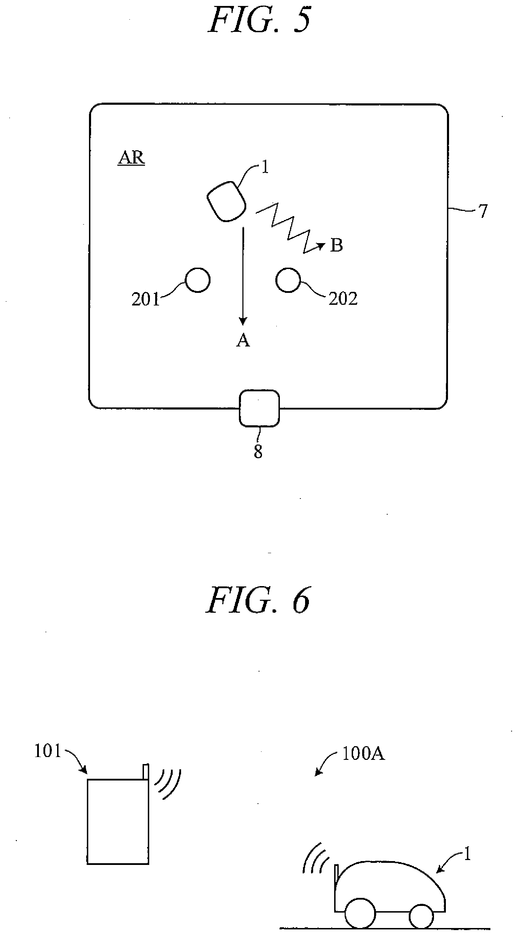

The operation of the emotion improving apparatus 100 according to the present embodiment will be described more specifically. FIG. 5 is a plan view of the working area AR showing an example of the operation of the emotion improving apparatus 100. As shown in FIG. 5, if a conversation between multiple users 201, 202 in the working area AR is detected, the process in FIG. 4 is started. If the users are quarrelling with each other, the working machine 1 determines that the users have predetermined uncomfortable emotions (e.g., angry emotions) (step S3). For this reason, the working machine 1 selects an operation having the highest priority, for example, a travel operation in which the working machine 1 traverses between the users 201, 202, as a predetermined improvement operation (step S4). The working machine 1 then travels so as to traverse between the users 201, 202, as shown by an arrow A in FIG. 5, while grasping the positions of the users 201, 202 using the camera 21 or the like (step S5).

When the working machine 1 traverses between the users 201, 202 as described above, the attention of the users 201, 202 is directed to the working machine 1, that is, the operation of the working machine 1 gives the users 201, 202 an opportunity for restoring calm. Thus, the users 201, 202 stop quarrelling with each other, improving the levels of the uncomfortable emotions thereof to the predetermined value or more.

On the other hand, if the levels of the emotions of the users have not been improved to the predetermined value or more although the predetermined improvement operation has been performed, the working machine 1 lowers the priority of that improvement operation and selects another improvement operation (step S7 → step S4). For example, the working machine 1 selects an improvement operation in which it travels in a zig-zag pattern around the users 201, 202, as an operation having the second highest priority. Thus, the working machine 1 travels in a zig-zag pattern around the users 201, 202, as shown by the arrow B in FIG. 5, while grasping the positions of the users 201, 202 using the camera 21 or the like. Then, if the uncomfortable emotions of the users 201, 202 are improved, the working machine 1 ends the improvement operation; if the uncomfortable emotions are not improved, the working machine 1 lowers the priority of the selected improvement operation and performs another improvement operation.

The present embodiment can produce the following advantageous effects:
(1) The emotion improving apparatus 100 for improving uncomfortable emotions of users using the working machine 1 includes the biological information acquiring unit 20 that acquires biological information of the users, the uncomfortable emotion determining unit 11 that determines whether predetermined uncomfortable emotions are occurring to the users, on the basis of the biological information acquired by the biological information acquiring unit 20, and the working machine controlling unit 15 that if the uncomfortable emotion determining unit 11 determines that the predetermined uncomfortable emotions are occurring, controls the working machine 1 so that the working machine 1 performs a predetermined improvement operation for improving the uncomfortable emotions, toward the users (FIGs. 2A, 2B). According to this configuration, the working machine can be used to improve uncomfortable emotions of the users, in particular, uncomfortable emotions caused by an interaction between the users (e.g., angry emotions caused by a quarrel between the users).
(2) The uncomfortable emotion determining unit 11 includes the emotion estimating unit 12 that estimates the emotions of the users on the basis of the biological information acquired by the biological information acquiring unit 20 (FIG. 2B). The uncomfortable emotion determining unit 11 obtains the levels of the uncomfortable emotions of the users on the basis of the emotions estimated by the emotion estimating unit 12 and, if the obtained levels reach the predetermined value, determines that predetermined uncomfortable emotions are occurring. By obtaining the levels of the uncomfortable emotions of the users as described above, a suitable improvement operation corresponding to the levels of the uncomfortable emotions can be performed.
(3) The emotion improving apparatus 100 further includes the operation storing unit 13 that stores multiple improvement operations, which are predetermined candidate improvement operations, with priorities and the operation selecting unit 14 that selects an improvement operation having a high priority from among the improvement operations stored in the operation storing unit 13 (FIG. 2B). If the uncomfortable emotion determining unit 11 determines that predetermined uncomfortable emotions are occurring, the working machine controlling unit 15 controls the working machine 1 so that the working machine 1 performs the improvement operation selected by the operation selecting unit 14. By assigning the priorities to the improvement operations as described above, an improvement operation expected to be most effective can be selected if it is determined that predetermined uncomfortable emotions are occurring. Thus, the uncomfortable emotions of the users can be effectively improved.
(4) The emotion improving apparatus 100 further includes the effect measuring unit 16 that measures the degrees of improvement of the uncomfortable emotions of the users when the working machine 1 performs the operation selected by the operation selecting unit 14 (FIG. 2B). If it is determined that the degrees of improvement of the uncomfortable emotions measured by the effect measuring unit 16 are less than the predetermined value, the operation selecting unit 14 lowers the priority of the selected improvement operation and selects another improvement operation. As seen above, the priority of the improvement operation is updated in accordance with the degrees of improvement of the uncomfortable emotions, allowing the working machine 1 to select the most suitable improvement operation. Even if a predetermined improvement operation is most effective for a particular user, the effectiveness may degrade with time. According to the present embodiment, priorities of the improvement operations are updated in accordance with the degrees of improvement of the uncomfortable emotions at any time, allowing the most suitable improvement operation to be always selected.
(5) The biological information acquiring unit 20 includes the camera 21, microphone 22, and thermography 23 disposed on the working machine 1 (FIG. 2A). Thus, the facial expressions, motions, speeches, and body temperatures (facial surface temperatures) of the users can be recognized, and the levels of the uncomfortable emotions of the users used as the precondition of an improvement operation can be favorably estimated.
(6) The working machine 1 comprises a lawn mower that mows the lawn while autonomously traveling within the working area AR (FIG. 1). Since the working machine 1 thus configured works near the users, the users feel familiar with the working machine 1. For this reason, uncomfortable emotions of the users can be effectively improved through the working machine 1.
(7) The working machine 1 is a moving working machine that performs predetermined work while autonomously traveling within the working area AR (FIG. 1). The biological information acquiring unit 20 acquires biological information of multiple users. The uncomfortable emotion determining unit 11 determines whether predetermined uncomfortable emotions are occurring due to an interaction between the users, on the basis of the biological information of the users acquired by the biological information acquiring unit 20. If the uncomfortable emotion determining unit 11 determines that predetermined uncomfortable emotions are occurring, the working machine controlling unit 15 controls the working machine 1 so that the working machine 1 travels around the users (e.g., between the users 201, 202) (FIG. 5). Thus, the attention of the users is directed to the working machine 1 and therefore a quarrel between the users, or the like can be effectively suppressed.
(8) The emotion improving method for improving uncomfortable emotions of users using the working machine 1 includes acquiring biological information of the users (step S1), determining whether predetermined uncomfortable emotions are occurring to the users, on the basis of the acquired biological information (step S3), and if it is determined that the predetermined uncomfortable emotions are occurring, controlling the working machine 1 so that the working machine 1 performs a predetermined improvement operation for improving the uncomfortable emotions, toward the users (step S5). Thus, the uncomfortable emotions of the users, such as anger, can be effectively improved.

The above embodiment can be modified into various forms, and modifications will be described below. FIG. 6 is a diagram showing an emotion improving apparatus 100A as a modification of FIG. 1. In FIG. 1, the working machine 1 alone forms the emotion improving apparatus 100; in FIG. 6, a working machine 1 and a processor 101 form the emotion improving apparatus 100A. The processor 101 includes an ECU, a memory unit, a communication unit, and the like, and the working machine 1 and the processor 101 are configured to be able to wirelessly communicate with each other through the communication unit. According to this configuration, the ECU 10 of the working machine 1 is able to obtain data from the processor 101 as necessary. As seen above, part of the ECU 10 can be disposed on the processor 101, simplifying the configuration of the ECU 10.

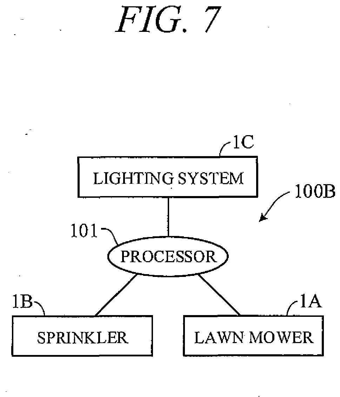

While, in the above embodiment, the single working machine 1 configures the emotion improving apparatus 100, multiple working machines may configure an emotion improving apparatus. FIG. 7 is a block diagram conceptually showing an example of such an emotion improving apparatus. In FIG. 7, the lawn mower (represented by 1A) described above as the working machine 1 and a sprinkler (represented by 1B) are connected to a processor 101 so as to be able to communicate therewith wirelessly or by wire. The sprinkler 1B is connected to a water supply through piping and is able to sprinkle water on a predetermined outdoor area through a nozzle in response to the activation of a solenoid valve (working actuator) that connects or disconnects the water supply and nozzle. A lighting system 1C for illuminating the predetermined outdoor area is also connected to the processor 101.

In an emotion improving apparatus 100B thus configured, one or more (e.g., all) of the lawn mower 1A, sprinkler 1B and lighting system 1C are provided with a biological information acquiring unit 20. The processor 101 determines whether predetermined uncomfortable emotions (e.g., angry emotions) are occurring to the users, on the basis of biological information acquired by the biological information acquiring unit 20. If it determines that predetermined uncomfortable emotions are occurring, the processor 101 controls the lawn mower 1A, sprinkler 1B, and lighting system 1C so that these devices perform a predetermined operation for improving the uncomfortable emotions, toward the users. For example, instead of or in addition to the operation of the lawn mower 1A, the processor 101 controls the solenoid valve of the sprinkler 1B so that the sprinkler 1B sprinkles water. In this case, the sprinkler 1B may be caused to sprinkle water considering the direction of the Sun so that a rainbow is produced. Also, the processor 101 may control the lighting system 1C so that the lighting system 1C blinks. The sprinkler 1B alone, for example, may be used as a working machine.

As described above, the working machine 1 may be non-self-propelled (fixed) rather than self-propelled (movable). Also, the configuration of the working machine is not limited to that described above. For example, the working machine may be a movable working machine that includes a movable arm and a movable hand and is able to grasp plants or the like. This working machine may be used to gather plants or the like to improve uncomfortable emotions of users. That is, the predetermined operations for improving the uncomfortable emotions are not limited to those described above, and the working machine controlling unit may be of any type as long as it controls the working machine so that, if it is determined that predetermined uncomfortable emotions are occurring, the working machine performs a predetermined operation for improving the uncomfortable emotions, toward the users.

While, in the above embodiment, the camera 21 as an imaging device, the microphone 22 as a sound collector, and the thermography 23 as a temperature imaging device detect states of users, which change in accordance with the work satisfaction level, a biological information acquiring unit is not limited to this configuration. For example, not all of the imaging device, sound collector, and temperature imaging device but at least one of these may acquire biological information. Also, another type of device (wearable device, etc.) may acquire the same biological information as that described above or another type of biological information (e.g., pulse or heart rate). The biological information acquiring unit may be disposed on an entity (e.g., the wall of a building) other than the working machine.

In the above embodiment, the emotion estimating unit 12 estimates the emotions of the users on the basis of the biological information acquired by the biological information acquiring unit 20, and the uncomfortable emotion determining unit 11 obtains the levels of the uncomfortable emotions of the users on the basis of the estimated emotions and, if the obtained levels reach the predetermined value (predetermined negative value), determines that predetermined uncomfortable emotions (e.g., uncomfortable emotions of angry) are occurring. However, an uncomfortable emotion determining unit may simply determine whether predetermined uncomfortable emotions are occurring to the users, without obtaining the levels of the uncomfortable emotions. Accordingly, an emotion estimating unit may be omitted.

In the above embodiment, multiple improvement operations, which are predetermined candidate operations for improving uncomfortable emotions, are stored with priorities in the operation storing unit 13, and the operation selecting unit 14 selects an operation having a high priority from among the stored operations. However, for example, a given operation may be always performed in order to improve uncomfortable emotions. Accordingly, an operation storing unit and an operation selecting unit may be omitted. While, in the above embodiment, the effect measuring unit 16 measures the degrees of improvement of the uncomfortable emotions of the users, an effect measuring unit may be omitted.

The above description is only an example, and the present invention is not limited to the above embodiment and modifications, unless impairing features of the present invention. The above embodiment can be combined as desired with one or more of the above modifications. The modifications can also be combined with one another.

### REFERENCE SIGNS LIST

1 working machine, 11 uncomfortable emotion determining unit, 12 emotion estimating unit, 13 operation storing unit, 14 operation selecting unit, 15 working machine controlling unit, 16 effect measuring unit, 20 biological information acquiring unit, 21 camera, 22 microphone, 23 thermography, 100, 100A, 100B emotion improving apparatus

## Claims

1. An emotion improving apparatus (100) for improving an uncomfortable emotion of a user of a working machine (1), comprising:
the working machine (1) comprising at least one of a lawn mower (1) configured to perform a lawn-mowing work while autonomously traveling in a working area and a sprinkler (1);
a biological information acquiring unit (20) configured to acquire a biological information of the user;
an uncomfortable emotion determining unit (11) configured to determine whether a predetermined uncomfortable emotion occurs to the user based on the biological information acquired by the biological information acquiring unit (20); and
a working machine controlling unit (15) configured to control the working machine (1) to perform a predetermined operation for improving the uncomfortable emotion to the user when it is determined by the uncomfortable emotion determining unit (11) that the predetermined uncomfortable emotion occurs.

2. The emotion improving apparatus (100) according to claim 1, wherein
the uncomfortable emotion determining unit (11) has an emotion estimating unit (12) configured to estimate an emotion of the user based on the biological information acquired by the biological information acquiring unit (20), and is configured to determine a level of the uncomfortable emotion of the user based on the emotion estimated by the emotion estimating unit (12), and configured to determine that the predetermined uncomfortable emotion occurs when the determined level reaches a predetermined value.

3. The emotion improving apparatus (100) according to claim 1 or 2, further comprising:
an operation storing unit (13) configured to store multiple operations, which are candidates of the predetermined operation, with priorities; and
an operation selecting unit (14) configured to select an operation with high priority from among the multiple operations stored in the operation storing unit (13), wherein
the working machine controlling unit (15) is configured to control the working machine to perform the operation selected by the operation selecting unit (14) when it is determined by the uncomfortable emotion determining unit (11) that the predetermined uncomfortable emotion occurs.

4. The emotion improving apparatus (100) according to claim 3, further comprising
an effect measuring unit (16) configured to measure a degree of improvement of the uncomfortable emotion of the user when the working machine (1) performs the operation selected by the operation selecting unit (14), wherein
the operation selecting unit (14) is configured to lower a priority of the operation selected by the operation selecting unit (14) and configured to select another operation, when it is determined that the degree of improvement of the uncomfortable emotion measured by the effect measuring unit (16) is less than a predetermined value.

5. The emotion improving apparatus (100) according to any one of claims 1 to 4, wherein
the biological information acquiring unit (20) has at least one of an imaging device (21), a sound collecting device (22) and a temperature imaging device (23) installed on the working machine (1).

6. The emotion improving apparatus (100) according to any one of claims 1 to 5, wherein
the biological information acquiring unit (20) is configured to acquire the biological information of multiple users,
the uncomfortable emotion determining unit (11) is configured to determine whether the predetermined uncomfortable emotion occurs to the multiple users based on the biological information of the multiple users acquired by the biological information acquiring unit (20), and
the working machine controlling unit (15) is configured to control the working machine (1) to travel around the multiple users when it is determined by the uncomfortable emotion determining unit (11) that the predetermined uncomfortable emotion occurs.

7. An emotion improving method (100) for improving an uncomfortable emotion of a user of a working machine (1), wherein the working machine (1) comprises at least one of a lawn mower (1) configured to perform a lawn-mowing work while autonomously traveling in a working area and a sprinkler (1), comprising:
acquiring, by means of an biological information acquiring unit (20), a biological information of the user (SI);
determining, by means of an uncomfortable emotion determining unit (11), whether a predetermined uncomfortable emotion occurs to the user based on the biological information acquired (S3); and
controlling, by means of a working machine controlling unit (15), the working machine (1) to perform a predetermined operation for improving the uncomfortable emotion to the user when it is determined that the predetermined uncomfortable emotion occurs (S5).

8. The emotion improving method (100) according to claim 7, wherein
the determining (S3) includes estimating an emotion of the user based on the biological information acquired (S2), determining a level of the uncomfortable emotion of the user based on the emotion estimated, and determining that the predetermined uncomfortable emotion occurs when the determined level reaches a predetermined value.

9. The emotion improving method (100) according to claim 7 or 8, further comprising:
storing multiple operations, which are candidates of the predetermined operation, with priorities; and
selecting an operation with high priority from among the multiple operations stored, wherein
the controlling includes controlling the working machine (1) to perform the operation selected when it is determined that the predetermined uncomfortable emotion occurs.

10. The emotion improving method (100) according to claim 9, further comprising
measuring a degree of improvement of the uncomfortable emotion of the user when the working machine (1) performs the operation selected, wherein
the selecting includes lowering a priority of the operation selected and selecting another operation, when it is determined that the degree of improvement of the uncomfortable emotion measured is less than a predetermined value.

11. The emotion improving method (100) according to any one of claims 7 to 10, wherein
the acquiring (S1) includes at least one of capturing an image of the user, acquiring a speech uttered by the user, and detecting a temperature of the user.

12. The emotion improving method (100) according to any one of claims 7 to 11, wherein
the acquiring includes acquiring the biological information of multiple users,
the determining including determining whether the predetermined uncomfortable emotion occurs to the multiple users based on the biological information of the multiple users acquired, and
the controlling including controlling the working machine (1) to travel around the multiple users when it is determined that the predetermined uncomfortable emotion occurs.

## Patentansprüche

1. Emotionsverbesserungsvorrichtung (100) zum Verbessern einer unangenehmen Emotion eines Benutzers einer Arbeitsmaschine (1), aufweisend:
die Arbeitsmaschine (1), bestehend aus zumindest einer der Komponenten Rasenmäher (1) und Sprenger (1), wobei der Rasenmäher so ausgebildet ist, dass er eine Rasenmäharbeit durchführt, während er sich autonom in einem Arbeitsbereich bewegt;
eine biologische Informationserfassungseinheit (20), die ausgebildet ist, um eine biologische Information des Benutzers zu erfassen;
eine Einheit (11) zum Erkennen einer unangenehmen Emotion, ausgebildet um, basierend auf der biologischen Information, die von der Einheit (20) zum Erfassen biologischer Information erfasst wurde, festzustellen, ob eine vordefinierte unangenehme Emotion bei dem Benutzer vorliegt; und
eine Arbeitsmaschinen-Steuereinheit (15), die so konfiguriert ist, dass sie die Arbeitsmaschine (1) so steuert, dass diese eine vorgegebene Aktion zum Verbessern der unangenehmen Emotion beim Benutzer durchführt, wenn durch die Einheit (11) zum Erkennen einer unangenehmen Emotion festgestellt wird, dass die vordefinierte unangenehme Emotion vorliegt.

2. Emotionsverbesserungsvorrichtung (100) nach Anspruch 1, wobei
die Einheit (11) zum Erkennen einer unangenehmen Emotion eine Emotionsschätzeinheit (12) aufweist, die so ausgebildet ist, dass sie eine Emotion des Benutzers auf der Grundlage der von der Einheit (20) zum Erfassen biologischer Informationen erfassten biologischen Informationen schätzt, und so ausgebildet ist, dass sie einen Pegel der unangenehmen Emotion des Benutzers auf der Grundlage der von der Emotionsschätzeinheit (12) geschätzten Emotion erfasst, und so ausgebildet ist, dass sie entscheidet, dass die vordefinierte unangenehme Emotion vorliegt, wenn der erfasste Pegel einen vorgegebenen Wert erreicht.

3. Emotionsverbesserungsvorrichtung (100) nach Anspruch 1 oder 2, ferner aufweisend
eine Aktionsspeichereinheit (13), die so ausgebildet ist, dass sie mehrere Aktionen, die der vorgegebenen Aktion zugehörig sind, Prioritäts-bezogen speichert; und
eine Aktionsauswahleinheit (14), die ausgebildet ist, um eine Aktion mit hoher Priorität aus den mehreren in der Aktionsspeichereinheit (13) gespeicherten Aktionen auszuwählen, wobei
die Arbeitsmaschinen-Steuereinheit (15) so konfiguriert ist, dass sie die Arbeitsmaschine so steuert, dass sie die von der Aktionsauswahleinheit (14) ausgewählte Aktion durchführt, wenn von der Einheit (11) zum Erkennen einer unangenehmen Emotion festgestellt wird, dass die vordefinierte unangenehme Emotion vorliegt.

4. Emotionsverbesserungsvorrichtung (100) nach Anspruch 3, ferner aufweisend
eine Effektmesseinheit (16), die so ausgebildet ist, dass sie einen Grad der Verbesserung der unangenehmen Emotion des Benutzers misst, wenn die Arbeitsmaschine (1) die von der Aktionsauswahleinheit (14) ausgewählte Aktion durchführt, wobei
die Aktionsauswahleinheit (14) so ausgebildet ist, dass sie eine Priorität der von der Aktionsauswahleinheit (14) ausgewählten Aktion herabsetzt und so ausgebildet ist, dass sie eine andere Aktion auswählt, wenn festgestellt wird, dass der von der Effektmesseinheit (16) gemessene Grad der Verbesserung der unangenehmen Emotion kleiner als ein vorgegebener Wert ist.

5. Emotionsverbesserungsvorrichtung (100) nach einem der Ansprüche 1 bis 4, wobei
die Einheit (20) zum Erfassen biologischer Informationen zumindest eine der Komponenten Bildgebungseinrichtung (21), Schallerfassungseinrichtung (22) und Temperaturerfassungseinrichtung (23) aufweist, welche an der Arbeitsmaschine (1) vorgesehen sind.

6. Emotionsverbesserungsvorrichtung (100) nach einem der Ansprüche 1 bis 5, wobei
die Einheit (20) zum Erfassen biologischer Informationen ausgebildet ist, um die biologischen Informationen mehrerer Benutzer zu erfassen,
die Einheit (11) zum Erkennen einer unangenehmen Emotion ausgebildet ist, um festzustellen, ob die vordefinierte unangenehme Emotion bei den mehreren Benutzern vorliegt, basierend auf den biologischen Informationen der mehreren Benutzer, die von der Einheit (20) zum Erfassen biologischer Informationen erfasst werden, und
die Arbeitsmaschinen-Steuereinheit (15) so konfiguriert ist, dass sie die Arbeitsmaschine (1) so steuert, dass sie um die mehreren Benutzer herumfährt, wenn von der Einheit (11) zum Erkennen einer unangenehmen Emotion festgestellt wird, dass die vordefinierte unangenehme Emotion vorliegt.

7. Emotionsverbesserungsverfahren (100) zum Verbessern einer unangenehmen Emotion des Benutzers einer Arbeitsmaschine (1), wobei die Arbeitsmaschine (1) zumindest eine der Komponenten Rasenmäher (1) und Sprenger (1) aufweist, wobei der Rasenmäher so ausgebildet ist, dass er eine Rasenmäharbeit durchführt, während er sich autonom in einem Arbeitsbereich bewegt, aufweisend die folgenden Schritte:
Erfassen einer biologischen Information des Benutzers (S1) mittels einer Einheit (20) zum Erfassen biologischer Informationen;
Feststellen, ob eine unangenehme Emotion bei dem Benutzer vorliegt, mittels einer Einheit (11) zum Erkennen einer unangenehmen Emotion, basierend auf der erfassten biologischen Information (S3)
Steuern der Arbeitsmaschine (1) mittels einer Arbeitsmaschinen-Steuereinheit (15), um eine vorgegebene Aktion zur Verbesserung der unangenehmen Emotion beim Benutzer durchzuführen, wenn festgestellt wird, dass die vordefinierte unangenehme Emotion vorliegt (S5).

8. Emotionsverbesserungsverfahren (100) nach Anspruch 7, wobei
das Feststellen (S3) das Schätzen einer Emotion des Benutzers basierend auf der erfassten biologischen Information (S2), das Erfassen eines Pegels der unangenehmen Emotion des Benutzers basierend auf der geschätzten Emotion und das Feststellen, dass die vordefinierte unangenehme Emotion vorliegt, wenn der erfasste Pegel einen vorgegebenen Wert erreicht, umfasst.

9. Emotionsverbesserungsverfahren (100) nach Anspruch 7 oder 8, ferner aufweisend die folgenden Schritte:
Prioritätsbezogenes Speichern mehrerer Aktionen, die der vorgegebenen Aktion zugehörig sind; und
Auswählen einer Aktion mit hoher Priorität aus den mehreren gespeicherten Aktionen, wobei
das Steuern das Steuern der Arbeitsmaschine (1) umfasst, um die ausgewählte Aktion durchzuführen, wenn festgestellt wird, dass die vordefinierte unangenehme Emotion vorliegt.

10. Emotionsverbesserungsverfahren (100) nach Anspruch 9, ferner aufweisend die Schritte:
Messen eines Grades der Verbesserung der unangenehmen Emotion des Benutzers, wenn die Arbeitsmaschine (1) die ausgewählte Aktion durchführt, wobei
das Auswählen das Herabsetzen einer Priorität der ausgewählten Aktion und das Auswählen einer anderen Aktion umfasst, wenn festgestellt wird, dass der gemessene Grad der Verbesserung der unangenehmen Emotion kleiner als ein vorgegebener Wert ist.

11. Emotionsverbesserungsverfahren (100) nach einem der Ansprüche 7 bis 10, wobei
das Erfassen (S1) zumindest eines der folgenden Komponenten umfasst: Erfassen eines Bildes des Benutzers, Erfassen einer Äußerung des Benutzers und Erfassen einer Temperatur des Benutzers.

12. Emotionsverbesserungsverfahren (100) nach einem der Ansprüche 7 bis 11, wobei
das Erfassen das Erfassen der biologischen Information von mehreren Benutzern umfasst,
das Bestimmen das Bestimmen einschließt, ob die vordefinierte unangenehme Emotion bei den mehreren Benutzern vorliegt, basierend auf der erfassten biologischen Information der mehreren Benutzer, und
das Steuern das Fahren der Arbeitsmaschine (1) um die mehreren Benutzer herum miteinschließt, wenn festgestellt wird, dass die vordefinierte unangenehme Emotion vorliegt.

## Revendications

1. Appareil d'amélioration de sentiment (100) pour améliorer un sentiment d'inconfort d'un utilisateur d'une machine de travail (1), comprenant :
la machine de travail (1) comprenant au moins l'un parmi une tondeuse à gazon (1) configurée pour effectuer un travail de tonte de gazon tout en se déplaçant de manière autonome dans une zone de travail, et un arroseur (1) ;
une unité d'acquisition d'informations biologiques (20) configurée pour acquérir des informations biologiques de l'utilisateur ;
une unité de détermination de sentiment d'inconfort (11) configurée pour déterminer si un sentiment d'inconfort prédéterminé se produit chez l'utilisateur sur la base des informations biologiques acquises par l'unité d'acquisition d'informations biologiques (20) ; et
une unité de commande de machine de travail (15) configurée pour commander la machine de travail (1) pour effectuer une opération prédéterminée pour améliorer le sentiment d'inconfort chez l'utilisateur, lorsqu'il est déterminé par l'unité de détermination de sentiment d'inconfort (11) que le sentiment d'inconfort prédéterminé se produit.

2. Appareil d'amélioration de sentiment (100) selon la revendication 1, dans lequel
l'unité de détermination de sentiment d'inconfort (11) a une unité de détermination de sentiment (12) configurée pour estimer un sentiment de l'utilisateur sur la base des informations biologiques acquises par l'unité d'acquisition d'informations biologiques (20), et est configurée pour déterminer un niveau du sentiment d'inconfort de l'utilisateur sur la base du sentiment estimé par l'unité de détermination de sentiment (12), et configurée pour déterminer que le sentiment d'inconfort prédéterminé se produit lorsque le niveau déterminé atteint une valeur prédéterminée.

3. Appareil d'amélioration de sentiment (100) selon la revendication 1 ou 2, comprenant en outre :
une unité de stockage d'opérations (13) configurée pour stocker des opérations multiples, qui sont des candidates de l'opération prédéterminée, avec des priorités ; et
une unité de sélection d'opérations (14) configurée pour sélectionner une opération avec une priorité élevée parmi les opérations multiples stockées dans l'unité de stockage d'opérations (13), dans lequel
l'unité de commande de machine de travail (15) est configurée pour commander la machine de travail pour effectuer l'opération sélectionnée par l'unité de sélection d'opérations (14) lorsqu'il est déterminé par l'unité de détermination de sentiment d'inconfort (11) que le sentiment d'inconfort prédéterminé se produit.

4. Appareil d'amélioration de sentiment (100) selon la revendication 3, comprenant en outre
une unité de mesure d'effet (16) configurée pour mesurer un degré d'amélioration du sentiment d'inconfort de l'utilisateur lorsque la machine de travail (1) effectue l'opération sélectionnée par l'unité de sélection d'opérations (14), dans lequel
l'unité de sélection d'opérations (14) est configurée pour abaisser une priorité de l'opération sélectionnée par l'unité de sélection d'opérations (14) et configurée pour sélectionner une autre opération, lorsqu'il est déterminé que le degré d'amélioration du sentiment d'inconfort mesuré par l'unité de mesure d'effet (16) est inférieur à une valeur prédéterminée.

5. Appareil d'amélioration de sentiment (100) selon l'une quelconque des revendications 1 à 4, dans lequel
l'unité d'acquisition d'informations biologiques (20) a au moins l'un parmi un dispositif d'imagerie (21), un dispositif de collecte de sons (22) et un dispositif d'imagerie de température (23) installés sur la machine de travail (1).

6. Appareil d'amélioration de sentiment (100) selon l'une quelconque des revendications 1 à 5, dans lequel
l'unité d'acquisition d'informations biologiques (20) est configurée pour acquérir les informations biologiques d'utilisateurs multiples,
l'unité de détermination de sentiment d'inconfort (11) est configurée pour déterminer si le sentiment d'inconfort prédéterminé se produit chez les utilisateurs multiples sur la base des informations biologiques des utilisateurs multiples acquises par l'unité d'acquisition d'informations biologiques (20), et
l'unité de commande de machine de travail (15) est configurée pour commander la machine de travail (1) pour se déplacer autour des utilisateurs multiples lorsqu'il est déterminé par l'unité de détermination de sentiment d'inconfort (11) que le sentiment d'inconfort prédéterminé se produit.

7. Procédé d'amélioration de sentiment (100) pour améliorer un sentiment d'inconfort d'un utilisateur d'une machine de travail (1), dans lequel la machine de travail (1) comprend au moins l'un parmi une tondeuse à gazon (1) configurée pour effectuer un travail de tonte de gazon tout en se déplaçant de manière autonome dans une zone de travail, et un arroseur (1), comprenant les étapes consistant à :
acquérir, au moyen d'une unité d'acquisition d'informations biologiques (20), des informations biologiques de l'utilisateur (S1) ;
déterminer, au moyen d'une unité de détermination de sentiment d'inconfort (11), si un sentiment d'inconfort prédéterminé se produit chez l'utilisateur sur la base des informations biologiques acquises (S3) ; et
commander, au moyen d'une unité de commande de machine de travail (15), la machine de travail (1) pour effectuer une opération prédéterminée pour améliorer le sentiment d'inconfort chez l'utilisateur lorsqu'il est déterminé que le sentiment d'inconfort prédéterminé se produit (S5).

8. Procédé d'amélioration de sentiment (100) selon la revendication 7, dans lequel
la détermination (S3) comporte l'estimation d'un sentiment de l'utilisateur sur la base des informations biologiques acquises (S2), la détermination d'un niveau du sentiment d'inconfort de l'utilisateur sur la base du sentiment estimé, et la détermination que le sentiment d'inconfort prédéterminé se produit lorsque le niveau déterminé atteint une valeur prédéterminée.

9. Procédé d'amélioration de sentiment (100) selon la revendication 7 ou 8, comprenant en outre :
le stockage d'opérations multiples, qui sont des candidates de l'opération prédéterminée, avec des priorités ; et
la sélection d'une opération avec une priorité élevée parmi les opérations multiples stockées, dans lequel
la commande comporte la commande de la machine de travail (1) pour effectuer l'opération sélectionnée lorsqu'il est déterminé que le sentiment d'inconfort prédéterminé se produit.

10. Procédé d'amélioration de sentiment (100) selon la revendication 9, comprenant en outre
la mesure d'un degré d'amélioration du sentiment d'inconfort de l'utilisateur lorsque la machine de travail (1) effectue l'opération sélectionnée, dans lequel
la sélection comporte l'abaissement d'une priorité de l'opération sélectionnée et la sélection d'une autre opération, lorsqu'il est déterminé que le degré d'amélioration du sentiment d'inconfort mesuré est inférieur à une valeur prédéterminée.

11. Procédé d'amélioration de sentiment (100) selon l'une quelconque des revendications 7 à 10, dans lequel
l'acquisition (S1) comporte au moins une parmi la capture d'une image de l'utilisateur, l'acquisition d'une parole prononcée par l'utilisateur, et la détection d'une température de l'utilisateur.

12. Procédé d'amélioration de sentiment (100) selon l'une quelconque des revendications 7 à 11, dans lequel
l'acquisition comporte l'acquisition des informations biologiques d'utilisateurs multiples,
la détermination comporte le fait de déterminer si le sentiment d'inconfort prédéterminé se produit chez les utilisateurs multiples sur la base des informations biologiques des utilisateurs multiples acquises, et
la commande comporte la commande de la machine de travail (1) pour se déplacer autour des utilisateurs multiples lorsqu'il est déterminé que le sentiment d'inconfort prédéterminé se produit.
